# EUROPEAN PATENT SPECIFICATION

(11) **EP 2 451 369 B1**
(45) Date of publication and mention of the grant of the patent: **16.07.2014**
(21) Application number: 10735106.6
(22) Date of filing: 14.06.2010
(51) Int. Cl.: A61B 17/70

(54) **VERTEBRAL STABILIZATION TRANSITION CONNECTOR**
ÜBERGANGSVERBINDER FÜR WIRBELSTABILISIERUNG
CONNECTEUR DE TRANSITION DE STABILISATION VERTÉBRALE

(30) Priority: 13.07.2009 US 501793
(43) Date of publication of application: 16.05.2012
(73) Proprietor: Zimmer Spine, Inc., Minneapolis, MN 55439 (US)
(72) Inventor: HESTAD, Hugh D., Minneapolis Minnesota 55410 (US); LANCIAL, Michael E., St. Louis Park Minnesota 55426 (US); FROEHLICH, Markus, CH-8362 Balterswil (CH); DARST RICE, Mark W., Minneapolis Minnesota 55417 (US)
(74) Representative: Manitz, Finsterwald & Partner GbR
(86) International application number: PCT/US2010/038504
(87) International publication number: WO 2011/008392

(56) References cited:
- WO-A1-00/57801
- WO-A1-2008/115622
- FR-A1- 2 767 669
- US-A1- 2008 140 133
- US-A1- 2008 215 095
- US-A1- 2009 177 231

## Description

### Technical Field

The disclosure is directed to a system for providing stabilization to one or more vertebrae of a spinal column. More particularly, the disclosure is directed to a system for providing dynamic stability or support to one or more spinal segments of a spinal column.

### Background

The spinal column is a highly complex system of bones and connective tissues that provides support for the body and protects the delicate spinal cord and nerves. The spinal column includes a series of vertebrae stacked one on top of the other, each vertebrae includes a vertebral body including an inner or central portion of relatively weak cancellous bone and an outer portion of relatively strong cortical bone. An intervertebral disc is situated between each vertebral body to cushion and dampen compressive forces experienced by the spinal column. A vertebral canal, called the foramen, containing the spinal cord and nerves is located posterior to the vertebral bodies. In spite of the complexities, the spine is a highly flexible structure, capable of a high degree of curvature and twist in nearly every direction. For example, the kinematics of the spine normally includes flexion, extension, rotation and lateral bending.

There are many types of spinal column disorders including scoliosis (abnormal curvature and twisting of the spine), kyphosis (abnormal forward curvature of the spine, usually in the thoracic spine), excess lordosis (abnormal backward curvature of the spine, usually in the lumbar spine), spondylolisthesis (forward displacement of one vertebra over another, usually in a lumbar or cervical spine) and other disorders caused by abnormalities, disease, or trauma, such as ruptured or slipped discs, degenerative disc disease, fractured vertebra, and the like. Patients that suffer from such conditions usually experience extreme and debilitating pain as well as diminished range of motion and nerve function. These spinal disorders may also threaten the critical elements of the nervous system housed within the spinal column.

One particular spinal fixation technique includes immobilizing portions of the spine of a patient by using connecting elements such as relatively rigid orthopedic spine rods that run generally parallel to the spine. Another technique utilizes less rigid connecting elements to provide a more dynamic stabilization of the affected regions of the spine. One example of such a spinal stabilization system is offered by the assignee of this invention, Zimmer Spine, Inc., as Dynesys®.

Installation of such systems may be accomplished, for example, by accessing the spine posterially and fastening hooks, bone screws, or other types of vertebral anchors to the pedicles or other bony structures of the appropriate vertebra. The vertebral anchors may be generally placed in a quantity of two per vertebrae, one on either side of the spinal cord, and serve as anchor points for the connecting elements.

It may be desirable for some spinal stabilization systems to have regions of more rigid stabilization and regions of more flexible stabilization. Accordingly, there is an ongoing need to provide alternative apparatus, devices, assemblies, systems and/or methods that can function to alleviate pain or discomfort, provide stability, such as dynamic stability, and/or restore a range of motion to a spinal segment of a spinal column.

Document US 2009/0177231 A1 discloses a vertebral stabilization system according to the preamble of claim 1.

Documents US 2008/0140133 A1, WO 00/57801 A1, US 2008/0215095 A1, FR 2 767 669 A1 and WO 2008/115622 A1 also disclose vertebral stabilization systems comprising coupled connection members, such as rigid rods and/or flexible members.

### Summary

The present invention relates to a device as claimed hereafter. Preferred embodiments of the invention are set forth in the dependent claims.

The disclosure is directed to several alternative designs and materials of manufacturing spinal fixation hardware, structures, and assemblies.

The disclosure is directed to embodiments of a spinal fixation assembly for connecting a rigid rod and a flexible cord, or other flexible member, along a region of the spinal column with a plurality of fasteners. The rigid rod and the flexible cord may be connected with a transition connector. The rigid rod includes a clam shell connector configured to receive the flexible cord. The clam shell connector includes a first segment and a second segment extending from the rigid rod. The first and second segments are connected to the rigid rod at one end and have a free end extending away from the rigid rod portion. The free end of the first segment is discontinuous from the free end of the second segment, providing a gap between the free end of the first segment and the free end of the second segment. The first segment and second segment of the clam shell connector are configured to be clamped around the flexible cord.

A method of securing a flexible member of a vertebral stabilization system to a rigid member of the flexible stabilization system using a transition connector is disclosed. A rigid rod having an end portion including a clam shell connector may be provided. The clam shell connector includes a first segment extending from an enlarged diameter portion of the rigid rod to a free end of the first segment and a second segment extending from the enlarged diameter portion of the rigid rod to a free end of the second segment. The free end of the first segment may be discontinuous with the free end of the second segment. The rigid rod may be provided with the clam shell connector in an open position in which the free end of the first segment is spread apart and spaced from the free end of the second segment. An end portion of the flexible cord, or other flexible member, may be positioned in the clam shell connector between the first segment and the second segment. A clamping force may then be exerted on the first segment and/or the second segment to urge the clam shell connector to a closed position in which the end portion of the flexible cord is clamped between the first segment and the second segment.

An example of a vertebral stabilization system includes a flexible member coupled to a rigid rod. The rigid rod has an end portion including a bore configured to receive an end portion of the flexible member. The bore further includes an area of increased diameter having a first end having a first diameter and a second end having a second diameter greater than the first diameter. A retainer ring is slidably disposed within the area of increased diameter. The retainer ring is configured to slide from the first end toward the second end as the portion of the flexible member is advanced into the bore. The retainer ring is biased toward the first end to maintain a radially compressive force on the flexible member.

The above summary of embodiments is not intended to describe each disclosed embodiment or every implementation of the invention.

### Brief Description of the Drawings

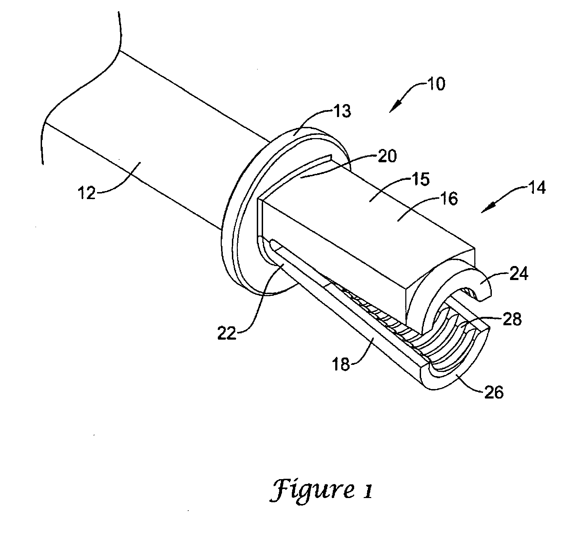

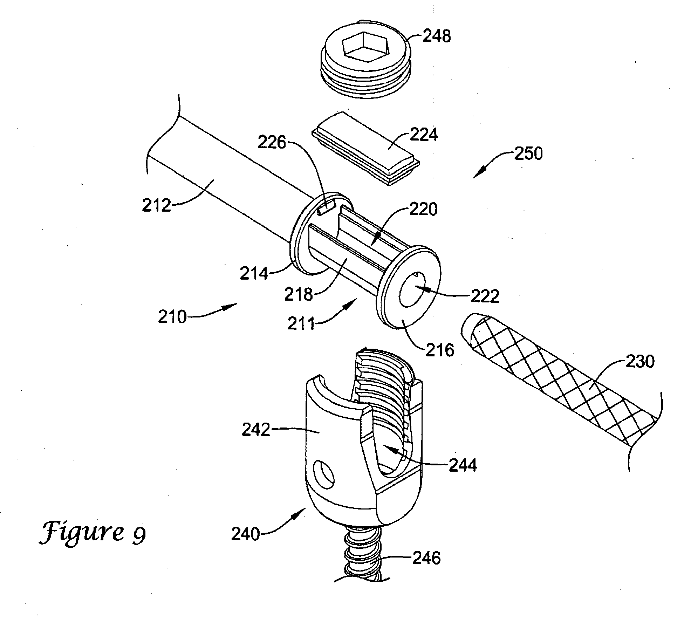

The invention is illustrated in the figures 1-8. The remaining figures 9-14 illustrate examples that are useful for understanding the invention.

The invention may be more completely understood in consideration of the following detailed description of various embodiments in connection with the accompanying drawings, in which:
FIG. 1 is a perspective view of an illustrative transition connector in a generally open position;
FIG. 2 is another perspective view of the illustrative transition connector of FIG. 1 during assembly of a flexible cord within the transition connector;
FIG. 2A is a side view of the transition connector and flexible cord of FIG. 2;
FIG. 3 is a perspective view of the illustrative transition connector of FIG. 1 in a generally closed position coupled to a flexible cord;
FIG. 3A is a side view of the transition connector and flexible cord of FIG. 3;
FIG. 4 is an axial cross-section of a vertebral stabilization system utilizing the illustrative transition connector of FIG. 1 coupled to a flexible cord;
FIG. 5 is a perspective view of another illustrative transition connector in a generally open position;
FIG. 6 is perspective view of the illustrative transition connector of FIG. 5 in a generally closed position coupled to a flexible cord;
FIG. 7 is an exploded view of components of an illustrative vertebral stabilization system utilizing a transition connector;
FIG. 8 is a perspective view of the components shown in FIG. 7 in an assembled configuration;
FIG. 9 is an exploded view of components of another illustrative vertebral stabilization system utilizing a transition connector (not part of the invention);
FIG. 10 is a perspective view of the components shown in FIG. 9 in an assembled configuration:
FIG. 10A is a longitudinal cross-sectional view of the assembled vertebral stabilization system of FIG. 10;
FIG. 11 is an exploded view of components of another illustrative vertebral stabilization system utilizing a transition connector (not part of the invention);
FIG. 12 is a perspective view of the components shown in FIG. 11 in an assembled configuration;
FIG. 12A is a longitudinal cross-sectional view of the assembled vertebral stabilization system of FIG. 12; and
FIGS. 13 and 14 are illustrative embodiments of an alternative transition connector (not part of the invention).

While the invention is amenable to various modifications and alternative forms, specifics thereof have been shown by way of example in the drawings and will be described in detail. It should be understood, however, that the intention is not to limit aspects of the invention to the particular embodiments described.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions, ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

As used herein, the terms "vertebral stabilization system", "vertebral stabilization construct" and similar terms encompass any type of construct extending between adjacent vertebrae regardless of its rigidity, flexibility or construction.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

Now referring to the drawings, FIG. 1 is a perspective view of an illustrative transition connector 10 for connecting a rigid rod construct and a flexible construct, to control relative motion of adjacent vertebrae along a region of a spinal column with a plurality of fasteners. In some embodiments, the flexible construct may be similar to the flexible cord of a vertebral stabliziataon system, as in the Dynesys® system offered by Zimmer Spine, Inc., the assignee of this invention. For example, the transition connector 10 may connect a rigid rod to a flexible member such as a flexible cord. The transition connector 10 may allow for a rigid spinal stabilization system to transition to a more flexible spinal stabilization system. The transition connector 10 may be used in conjunction with one or more vertebral fasteners as will be described in more detail below. The transition connector 10 may be formed from any biocompatible material, such as, but not limited to, titanium or stainless steel.

The transition connector 10 may include a first rigid rod portion 12 and a second clam shell connector 14. In some embodiments, the rigid rod portion 12, or portions thereof, may have a larger cross-section than the clam shell connector 14. In other embodiments, the rigid rod portion 12 may have a cross-section similar to or smaller than the clam shell connector 14. While the rigid rod portion 12 is shown as having a circular cross section, the rigid rod portion 12 may have a cross section of any desired shape, including, but not limited to: square, rectangular, polygonal, or elliptical. In some embodiments, the rigid rod portion 12 may further comprise an enlarged diameter portion or a flange portion 13 proximate the clam shell connector 14. The rigid rod portion 12 may be of any length necessary to extend between two, three, four, or more vertebrae of the spinal column.

In some embodiments, the rigid rod portion 12 and the clam shell connector 14 may be formed as a unitary structure. In other embodiments, the clam shell connector 14 may be fixedly attached to the rigid rod portion 12. For example, the clam shell connector 14 may be welded to the rigid rod portion 12 or may be attached using an adhesive. It is contemplated that if the clam shell connector 14 is fixedly attached to the rigid rod portion 12, the clam shell connector 14 and the rigid rod portion 12 may comprise different materials.

The clam shell connector 14 may have a first segment 16 and a second segment 18 extending from the rigid rod portion 12. For instance, the first and second segments 16, 18 may extend from the flange 13 of the rigid rod portion 12. While the clam shell connector 14 is illustrated as being formed of two segments, it is contemplated that the clam shell connector 14 may be formed of three, four, five, six, or more segments radially arranged and extending from the rigid rod portion 12.

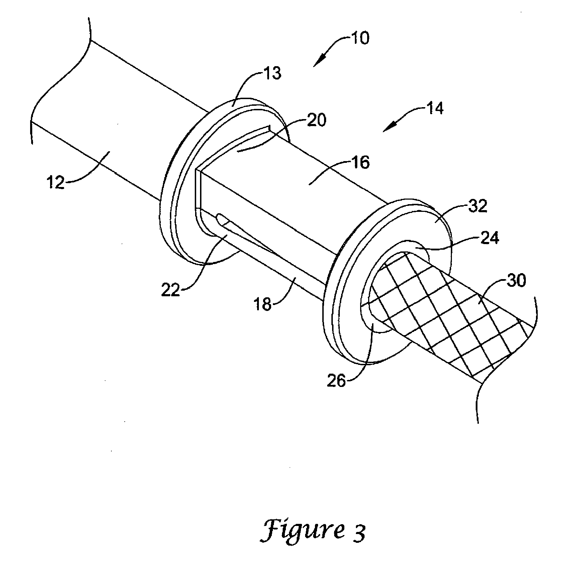

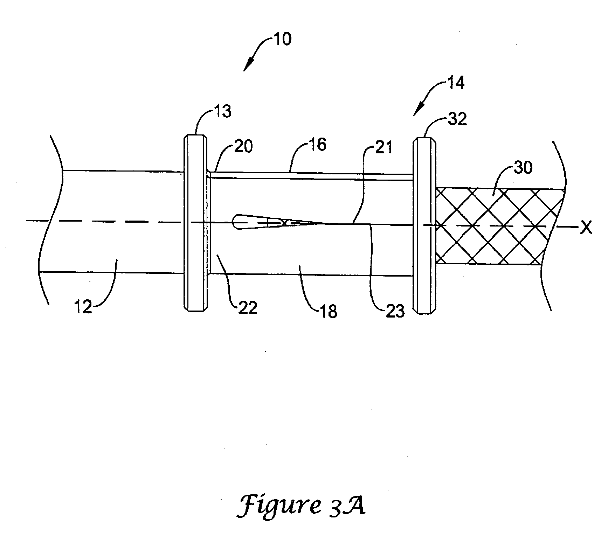

The first and second segments 16, 18 may each have a first end 20, 22 attached to the rigid rod portion 12 and a second free end 24, 26. The first and second segments 16, 18 may have a first generally open position where the free ends 24, 26 are discontinuous or spaced a distance from each other as shown in FIG. 1. The first and second segments 16, 18 may also have a generally closed position, as shown in FIG. 3, where the free ends 24, 26 are in close proximity to one another. The first segment 16 and second segment 18 may be shaped such that when they are in the closed position the segments 16, 18 collectively form a lumen therein. The lumen may extend from the first end 20, 22 to the second ends 24, 26 or a portion of the length between the first ends 20, 22 and second ends 24, 26. The first and second segments 16, 18 may have an inner surface 28 shaped to receive a flexible cord (not shown) or other flexible member. While the inner surface 28 is shown as having a generally concave shape, the inner surface 28 may be of any shape desired to accommodate any shaped cord or other flexible member such as, but not limited to, square, rectangular, polygonal, or elliptical. The inner diameter or cross-sectional dimension of the clam shell connector 14 when the first and second segments 16, 18 are in a generally closed position may be substantially the same as or smaller than an outer diameter or cross sectional dimension of the flexible cord or other flexible member.

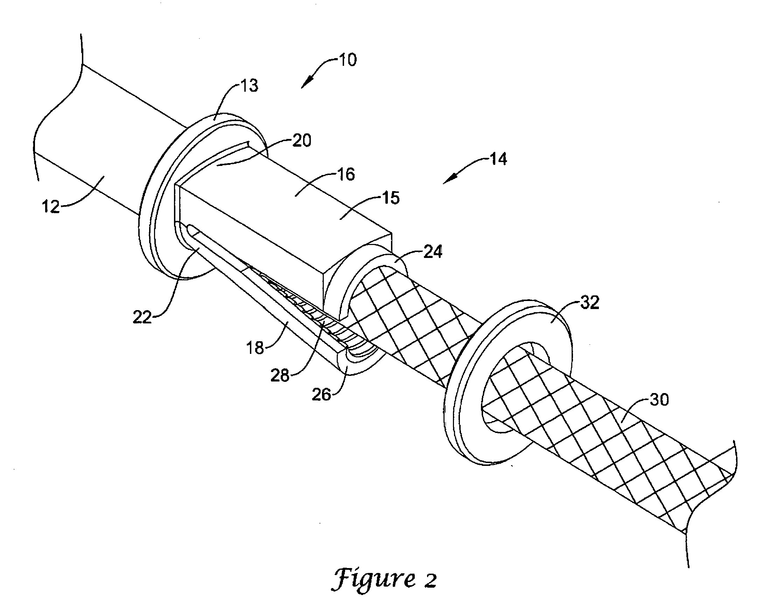

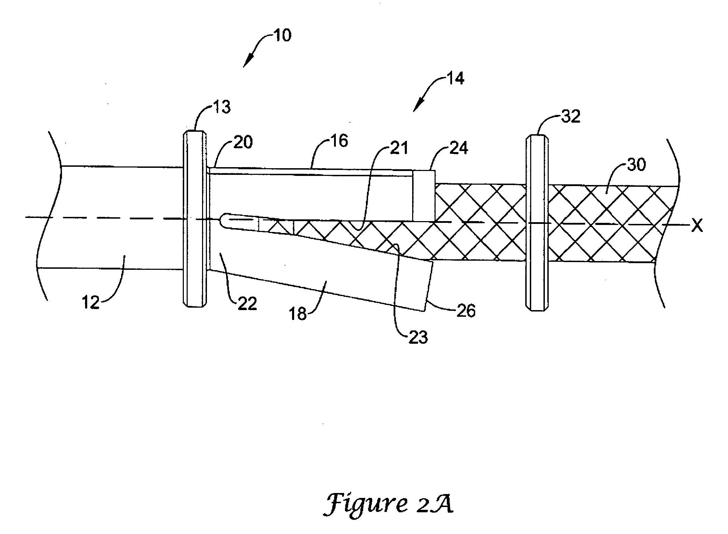

As can be seen in FIG. 1, in some embodiments the outer surfaces of the first and/or second segments 16, 18 of the clam shell connector 14 may comprise a different shape at the first attached end 20, 22 than at the second free end 24, 26. Furthermore, in some embodiments, the first segment 16 may have a different shape than the second segment 18. The first segment 16 may have a generally rectangular or square outer shape at the first end 20 including a flattened upper surface 15 and a circular shape towards the second free end 24. The flattened surface 15 may facilitate clamping the transition connector 10 within a vertebral fastener and the circular shape at the second free end 24 may facilitate maintaining the clam shell connector 14 in the closed position with a retaining ring as will be discussed in more detail with respect FIGS. 2 and 3. In some embodiments, the second segment 18 may have a generally round or convex outer shape configured to rest against the base of a generally U-shaped channel of a vertebral fastener.

However, the first and second segments 16, 18 may have any shape desired, such as, but not limited to square, rectangular, polygonal, or elliptical. Although each segment 16, 18 is shown as having a different shape, in some embodiments each segment 16, 18 may be substantially similar in shape if desired. The inner surface 28 of the first and second segments 16, 18 may be shaped to accommodate the shape of the cord regardless of the shape of the outer surfaces. While FIG. 1 shows one exemplary embodiment, the first and second segments 16, 18 of the clam shell connector 14 may have any combination of shapes desired.

Turning now to FIG. 2 which illustrates a perspective view of the transition connector 10 having a flexible member, shown as a flexible cord 30, disposed between the first and second segments 16, 18. While the flexible cord 30 is shown having a circular cross-section, the flexible cord 30 may have any cross-section desired such as, but not limited to, square, rectangular, polygonal, or elliptical. In one embodiment, the flexible cord 30 may be formed from polyethylene-terephthalate (PET), although it will be recognized that various other materials suitable for implantation within the human body and for providing stabilization of the spine while maintaining flexibility may be used. In other embodiments, the flexible cord 30 can be constructed of other flexible materials such as metal, polymeric materials, or combinations of flexible materials. The flexible cord 30 may be of any length necessary to extend between two, three, four, or more vertebrae of the spinal column. The flexible cord 30 may be positioned within the opening created between the first and second segments 16, 18 of the clam shell connector 14 when the first and second segments 16, 18 are in their open position.

The transition connector 10 may further comprise a retaining ring 32 which may be slidably disposed over the flexible cord 30 up to the segments 16, 18 of the clam shell connector 14. The retaining ring 32 may be configured to lock or maintain the first and second segments 16, 18 of the clam shell connector 14 in the generally closed position.

FIG. 2A is a side view of the assembly with the flexible cord 30 inserted into the lumen of the clam shell connector 14 with the first and second segments 16, 18 in the open position. As shown in FIG. 2A, in some embodiments, one of the first and second segments 16, 18 may extend non-parallel to the central longitudinal axis of the transition connector 10 (e.g., may extend non-parallel to the central longitudinal axis of the rigid rod portion 12), while the other of the first and second segments 16, 18 may extend parallel to the central longitudinal axis. For instance, the first segment 16 may extend parallel to the central longitudinal axis while the second segment 18 may extend at an oblique angle to the central longitudinal axis. As shown in FIG. 2A, in the open position, the contact edge 21 of the first segment 16 may be parallel to the central longitudinal axis, while the contact edge 23 of the second segment 18 may extend at an oblique angle to the central longitudinal axis.

Once the flexible cord 30 has been placed between the first and second segments 16, 18, a clamping force or other biasing means may be placed on the clam shell connector 14 to bias the first and/or second segments 16, 18 to the generally closed position (FIG. 3). For instance, a clamping force may be exerted on the segments 16, 18 to move the free ends 24, 26 toward one another. In some embodiments, the clamping force may cause the free end 24 of the first segment 16 to move toward the central longitudinal axis and/or may cause the free end 26 of the second segment 18 to move toward the central longitudinal axis.

As shown in FIG. 3A, in some embodiments, the clamping force may cause the free end 26 of the second segment 18 to move toward the central longitudinal axis (and thus, towards the free end 24 of the first segment 16) while the free end 24 of the first segment. 16 remains stationary. As the free end 26 of the second segment 18 moves toward the central longitudinal axis, the contact edge 23 of the second segment 18 moves toward the contact edge 21 of the first segment 16 such that the angle between the central longitudinal axis and the contact edge 23 decreases. In some instances, as shown in FIG. 3B, in the closed position, the contact edge 23 may abut or otherwise contact the contact edge 21 of the first segment 16. Thus, in the closed position, the second segment 18 may be moved such that the contact edge 23 may be parallel to the central longitudinal axis, in some instances.

When the clam shell connector 14 is in the generally closed position, the retaining ring 32 may be slid over the cord 30 and placed around the free ends 24, 26 to secure the clam shell connector 14 and to prevent the free ends 24, 26 from separating.

As previously discussed, the free ends 24, 26 may be circular in shape such that in the annular retaining ring 32 may freely slide over the free ends 24, 26 in the closed position with portions of the free ends 24, 26 disposed in the central opening of the retaining ring 32. In some embodiments, an inner diameter of the retaining ring 32 may be substantially the same as an outer diameter of the free ends 24, 26 in the closed position. In other embodiments, the inner diameter of the retaining ring 32 may be slightly less than the free ends 24, 26 of the clam shell connector 14 in the closed position creating a press fit between the components. In some embodiments, the retaining ring 32 may fit into a groove or channel extending circumferentially around the free ends 24, 26 of the clam shell connector 14.

The retaining ring 32 may be of any shape desired such that an inner surface of the ring 32 is configured to engage the free ends 24, 26 and maintain the clam shell connector 14 in the closed position. In some embodiments, the retaining ring 32 may be a discontinuous ring, such as a C-shaped ring, which can be expanded and/or contracted around the free ends 24, 26. For instance, the retaining ring 32 may be crimped around the free ends 24, 26 to secure the claim shell connector 14. In some embodiments, once the retaining ring 32 is disposed over the free ends 24, 26, the retaining ring 32 may be fixedly secured to the segments 16, 18 of the clam shell connector 14. The retaining ring 32 may, for example, be adhesively bonded or welded to the segments 16, 18 of the clam shell connector 14.

In some embodiments, the free ends 24, 26 of the clam shell connector 14 may further comprise a groove such that the retaining ring 32 may form a snap fit within the groove. In other embodiments, the free ends 24, 26 may taper such that the diameter at the free end is smaller than the first and second segments 16, 18. The retaining ring 32 may also comprise a taper configured to mate with the taper in the free ends 24, 26 of the clam shell connector 14.

As can be seen in FIG. 3, the retaining ring 32 may be disposed over the free ends 24, 26 such that the clam shell connector 14 is maintained in the closed position around the cord 30. In some embodiments, the outer diameter of the retaining ring 32 may be substantially the same as the cross-sectional dimension of the clam shell connector 14. In other embodiments, the outer diameter of the retaining ring 32 may be larger than the cross-sectional dimension of the clam shell connector 14. As shown in FIG. 3, the retaining ring 32 may have an outer diameter substantially equal to the outer diameter of the flange 13. In other embodiments, however, the outer diameter of the retaining ring 32 may be greater than or smaller than the outer diameter of the flange 13.

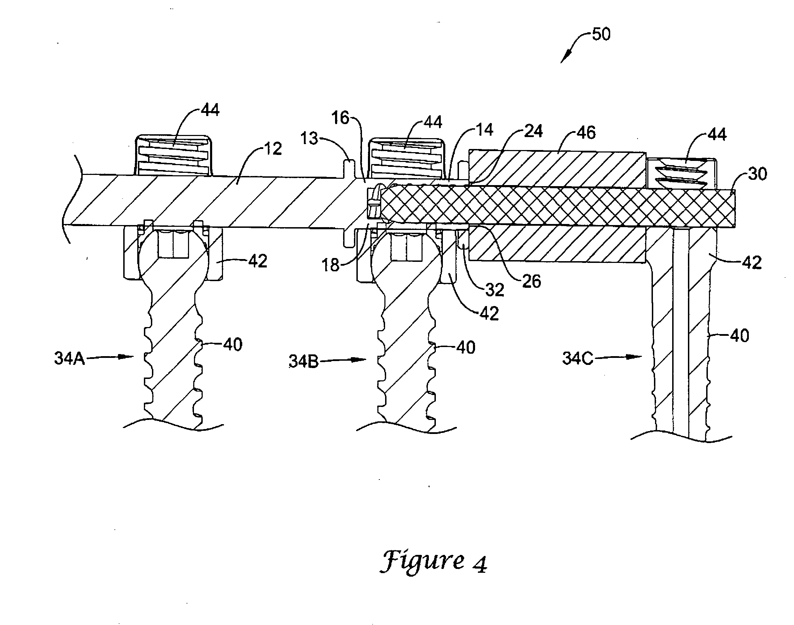

FIG. 4 shows an axial cross-section of a vertebral stabilization system 50 utilizing the illustrative transition connector 10 with three vertebral fasteners 34, illustrated as pedicle screws. The vertebral fasteners 34 may have a shank region 40, which in some embodiments may be a threaded region, for engaging a vertebra of the spinal column and a head portion 42, which in some embodiments may be an end region of the fastener 34 for receiving a stabilization device such as the rigid rod 12 or the flexible cord 30 of the vertebral stabilization system 50. In some embodiments, the head portion 42 of the fastener 34 may have a channel, such as a U-shaped channel, extending from a first side surface of the head portion 42 to a second side surface of the head portion 42 forming a saddle to receive a stabilization device. One or more of the vertebral fasteners 34, may be designed such that the head portion 42 is movable relative to the shank portion 40 to be lockable in one of a plurality of angular positions (i.e., polyaxial), while one or more of the vertebral fasteners 34 may be configured such that the shank region 40 is fixedly attached to the head portion 42 (i.e., monoaxial), as desired.

As can be seen in FIG. 4, the transition connector 10 may span more than one vertebral fastener 34. For example, the transition connector 10 may span between the first and second fasteners 34a, 34b. The rigid rod portion 12 of the transition connector 10 may be positioned in the channel of a first fastener 34a. The rigid rod portion 12 may be secured within the channel of the first vertebral fastener 34a by a set screw 44 or other locking means. In some embodiments, the rigid rod portion 12 may have a length such that the rigid rod portion 12 spans a plurality of fasteners (not explicitly shown).

A second end of the rigid rod portion 12 may abut or be positioned adjacent to a first side of a second fastener 34b. For instance, a side surface of the flange 13 may be positioned adjacent the head portion 42 of the second fastener 34b, such that the flange 13 faces the side surface of the head portion 42.

The clam shell connector 14 of the transition connector 10 may be positioned within the channel of the head portion 42 of the second fastener 34b. A first end of the flexible cord 30 may be disposed between the first and second segments 16, 18 of the clam shell connector 14. The clam shell connector 14 may extend past the second side of the second fastener 34b such that the free ends 24, 26 extend from the channel of the fastener 34b. The retaining ring 32 may be disposed over the free ends 24, 26 to maintain the clam shell connector 14 in the closed position. The retaining ring 32 may abut or may be positioned adjacent to a second side of the head portion 42 of the fastener 34b. The clam shell connector 14 may be secured within the channel of the fastener 34b by a set screw 44 or other locking means. The set screw 44 may engage the first segment 14 of the clam shell connector 14 to help maintain the clam shell connector 14 in the closed position by applying a clamping force against the clam shell connector 14. In some embodiments, the first segment 14 may have a flattened upper surface shown in FIG. 1 to enhance the area of contact between the clam shell connector 14 and a set screw 44 to secure the clam shell connector 14 within the channel of the fastener 34b.

The flexible cord 30 may extend out from the clam shell connector 14 towards a third fastener 34c. A second portion of the flexible cord 30 may be disposed within a channel of the third fastener 34c. The flexible cord 30 may be secured within the channel of the third fastener 34c by a set screw 44 or other locking means. In some embodiments, the flexible cord 30 may be sized such that it spans a plurality of fasteners (not explicitly shown).

A flexible spacer 46 may be disposed about the flexible cord 30 and disposed between a second side of the head portion 42 of the second fastener 34b and a first side of the head portion 42 of the third fastener 34c. In some embodiments, the flexible spacer 46 may include a central lumen through which the flexible cord 30 extends. In some embodiments, an end surface of the flexible spacer 46 may abut the retaining ring 32. In some embodiments, the flexible spacer 46 may be formed from polycarbonate urethane (PCU), although it will be recognized that various other materials suitable for implantation within the human body and for providing stabilization of the spine while maintaining flexibility may be used. In other embodiments, the flexible spacer 46 can be constructed of other flexible materials such as metal, polymeric materials, or combinations of flexible materials.

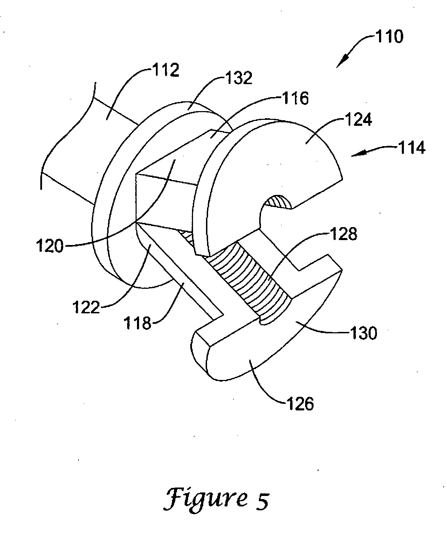

FIG. 5 shows another illustrative embodiment of a transition connector 110 in a generally open position. Transition connector 110 may have a rigid rod portion 112 and a clam shell connector 114 for connecting a rigid construct to a flexible construct, such as a cord (not shown). An enlarged portion, such as a flange 132, may be disposed between the rigid rod portion 112 and the clam shell connector 114. In some embodiments, the flange 132, rigid rod 112, and clam shell connector 114 may be formed as a unitary structure. Alternatively, the flange 132 may be fixedly attached to the rigid rod 112 and the clam shell connector 114 by any method known in the art, such as welding, brazing, or using an adhesive. It is contemplated that if the flange 132 is fixedly attached to the rigid rod portion 112 and the clam shell connector 114, the components may comprise different materials. The flange 132 is shown having a circular cross-section, however, the flange 132 may have any cross-section desired, such as, but not limited to square, rectangular, polygonal, or elliptical. The flange 132 may help maintain the transition connector 110 in a desired location when coupled to a fastener.

In some embodiments, the rigid rod portion 112 may have a larger cross-sectional dimension than the clam shell connector 114. In other embodiments, the rigid rod portion 112 may have a cross-sectional dimension substantially the same as or smaller than the clam shell connector 114. While the rigid rod portion 112 is shown as having a circular cross section, the rigid rod portion 112 may have a cross section of any desired shape, including, but not limited to: square, rectangular, polygonal, oval, or elliptical. The rigid rod portion 112 may be of any length necessary to extend between two, three, four, or more vertebrae of the spinal column.

The clam shell connector 114 may have a first segment 116 and a second segment 118 extending from the flange 132. While the clam shell connector 114 is illustrated as being formed of two segments, it is contemplated that the clam shell connector 114 may be formed of three, four, five, six, or more segments radially arranged and extending from the flange 132.

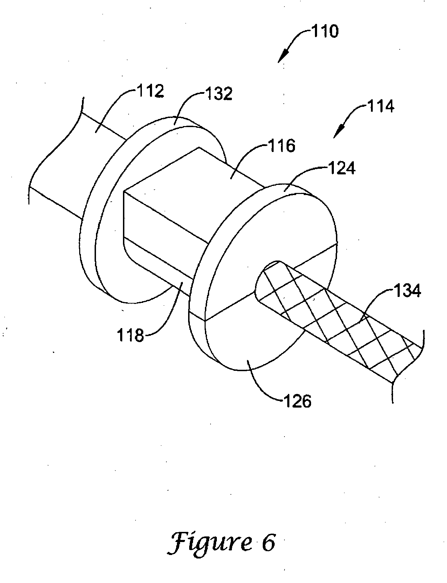

The first and second segments 116, 118 may each have a first end 120, 122 attached to the flange 132 and a second free end 124, 126. The first and second segments 116, 118 may have a first generally open position where the free ends 124, 126 are discontinuous or are spaced a distance from each other as shown in FIG. 5. The first and second segments 116, 118 may also have a generally closed position, as shown in FIG. 6, where the free ends 124, 126 are in close proximity to one another and/or in contact with one another. The free ends 124, 126 may have a diameter larger than the rest of first and second segments 116, 118 providing the free ends 124, 126 with a flange. The flange on the free ends 124, 126 may help maintain the transition connector 110 in a desired position when the clam shell connector 114 is positioned within the channel of a fastener. The free ends 124, 126 may have a generally circular cross-section (when in the closed position), as shown, however, the free ends 124, 126 (in the closed position) may have any cross-section desired, such as, but not limited to generally square, rectangular, polygonal, or elliptical.

As can be seen in FIG. 5, in some embodiments the outer surfaces of the first and/or second segments 116, 118 of the clam shell connector 114 may comprise a different shape at the first attached end 120, 122 than at the second free end 124, 126. Furthermore, in some embodiments, the first segment 116 may have a different shape than the second segment 118. For instance, the first segment 116 may have a generally rectangular or square outer shape at the first end 120, providing the first segment 116 with a flattened surface. The flattened surface may facilitate clamping the transition connector 110 within a vertebral fastener. In some embodiments, the second segment 118 may have a generally round or convex outer shape configured to rest against the base of a generally U-shaped channel of a vertebral faster.

However, the first and second segments 116, 118 may have any shape desired, such as, but not limited to square, rectangular, polygonal, or elliptical. Although each segment 116, 118 is shown as having a different shape, in some embodiments each segment 116, 118 may be substantially similar in shape. While FIG. 5 shows one exemplary embodiment, the first and second segments 116, 118 of the clam shell connector 114 may have any combination of shapes desired.

The first segment 116 and second segment 118 may be shaped such that when they are in the closed position the segments 116, 118 collectively form a lumen therein. The lumen may extend from the first ends 120, 122 to the second ends 124, 126 or a portion of the length between the first 120, 122 and second ends 124, 126. The first and second segments 116, 118 may have an inner surface 128 shaped to receive a flexible cord (not shown) or other flexible member. While the inner surface 128 is shown as having a generally concave shape, the inner surface 128 may be of any shape desired to accommodate any shaped cord or other flexible member such as, but not limited to, square, rectangular, polygonal, or elliptical. In some embodiments, the inner diameter or cross-sectional dimension of the clam shell connector 114 when the first and second segments 116, 118 are in a generally closed position may be substantially the same as the outer diameter or cross-sectional dimension of the flexible cord or other flexible member. In other embodiments, the inner diameter or cross-sectional dimension of the clam shell connector 114 in the closed position may be smaller than the outer diameter or cross-sectional dimension of the flexible cord or other flexible member. In some embodiments, the inner surface 128 may further comprise surface roughenings 130. The surface roughenings 130 may help maintain the cord within the clam shell connector 114 when the clam shell connector 114 is in the closed position. The surface roughenings 130 may be comprised of any mechanical gripping means such as, but not limited to, one or more threads, ribs, projecting grooves, teeth, and/or serrations or combination thereof.

FIG. 6 illustrates a perspective view of the transition connector 110 shown in FIG. 5 in the closed position. A flexible cord 134 may be disposed between the first segment 116 and the second segment 118 and extend away from the connector 110. Once the cord 134 has been placed between the first and second segments 116, 118, a clamping force or other biasing means may be placed on the first and second segments 116, 118 of the clam shell connector 114 to urge the first and second segments 116, 118 toward the generally closed position. In this embodiment a retaining ring may or may not be used to maintain the clam shell connector 114 in the closed position. For instance, the clam shell connector 114 may be held in the closed position by a clamping force applied to the vertebral fastener or may be biased towards the closed position.

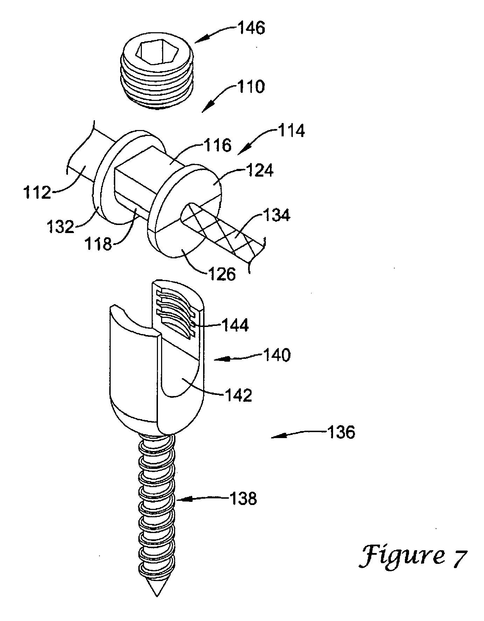

FIG. 7 illustrates an exploded view of the illustrative transition connector 110 shown in FIG. 5 in association with a vertebral fastener 136. The vertebral fastener 136 may have a shank portion 138 and a head portion 140. The shank portion 138 may be threaded to engage the vertebrae. The head portion 140 of the vertebral fastener 136 may include a channel 142, such as a U-shaped channel, extending from a first side surface of the head portion 140 to a second side surface of the head portion 140. The head portion 140 may further include an open end including a threaded region 144 for receiving a set screw 146 between the arms of the head portion 140. The set screw 146 may engage the first segment 116 of the clam shell connector 114 when the connector 110 is disposed within the channel 142 of the fastener 136. The second segment 118 of the clam shell connector 114 may be configured to substantially mate with the shape of the channel 142. For example, the channel 142 may be substantially U-shaped creating a concave lower surface. In this embodiment, the second segment 118 may be convex to rest in the concave portion of the U-shaped channel 142. The first segment 116 may have a flat top surface configured to provide an extended region of contact between the first segment 116 and the set screw 146 when the connector 110 is disposed and maintained in the channel 142 of the vertebral fastener 136. While the first and second segments 116, 118 are described as having particular shapes, it is contemplated that the first and second segments 116, 118 may be of any shape desired such as, but not limited to, square, rectangular, polygonal, elliptical, and/or circular.

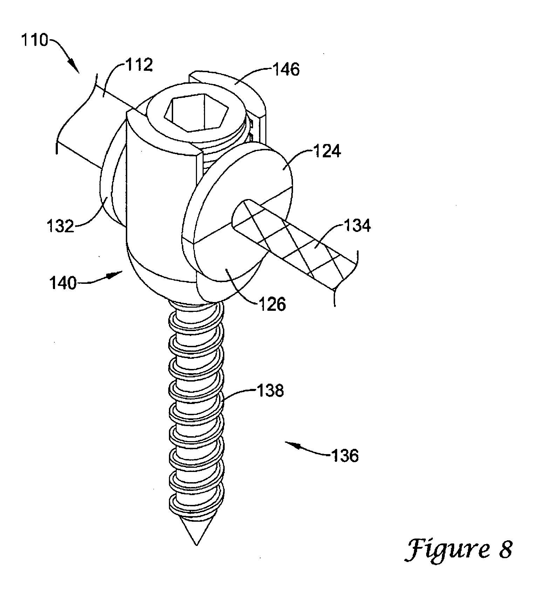

FIG. 8 illustrates a perspective view of the transition connector 110 assembled within the fastener 136. As can be seen, the clam shell connector 114 is disposed within the channel 142 of the fastener 136 such that the flange 132 is disposed adjacent to or abutting a first side surface of the head portion 140 of the fastener 136 and the flange of the free ends 124, 126 are disposed adjacent to or abutting a second side surface of the head portion 140 of the fastener 136. The flange 132 and the flange of the free ends 124, 126 may be sized such that they are larger than the channel 142 of the fastener 136. This may help maintain the clam shell connector 114 within the head 140 of the fastener 136 and/or may help prevent shifting of the vertebral stabilization system once it is placed within the body. The rigid rod portion 112 may be of any length necessary to extend between two, three, four, or more vertebrae of the spinal column. The flexible cord 134 may also extend from the clam shell connector 114 in a direction opposite the rigid rod portion 112. The cord 134 may be of any length necessary to extend between two, three, four, or more vertebrae of the spinal column.

The cord 134 may be provisionally secured or clamped in the clam shell connector 114 by a provisional clamping force applied to the cord 134 by the first and second segments 116, 118 being urged to the closed position. The cord 134 may be further secured or clamped in the clam shell connector 114 with a further clamping force applied to the segments 116, 118 by the set screw 146 when the set screw 146 is screwed into the threaded region 144 of the head portion 140. The securement of the clam shell connector 114 in the channel 142 of the head portion 140 with the set screw 146 may prevent the first segment 116 and the second segment 118 from spreading apart.

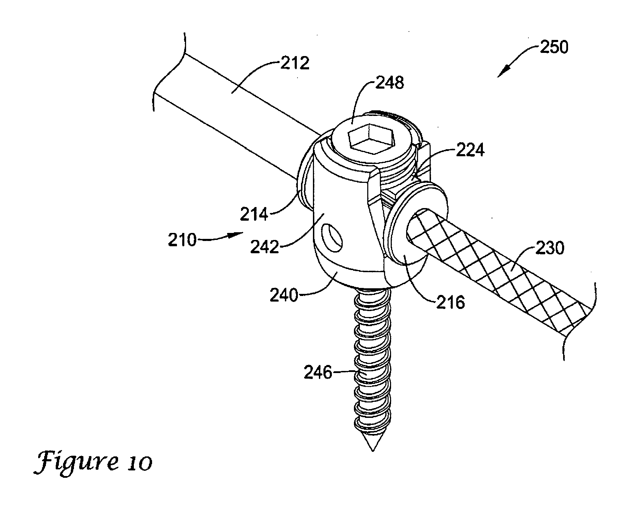

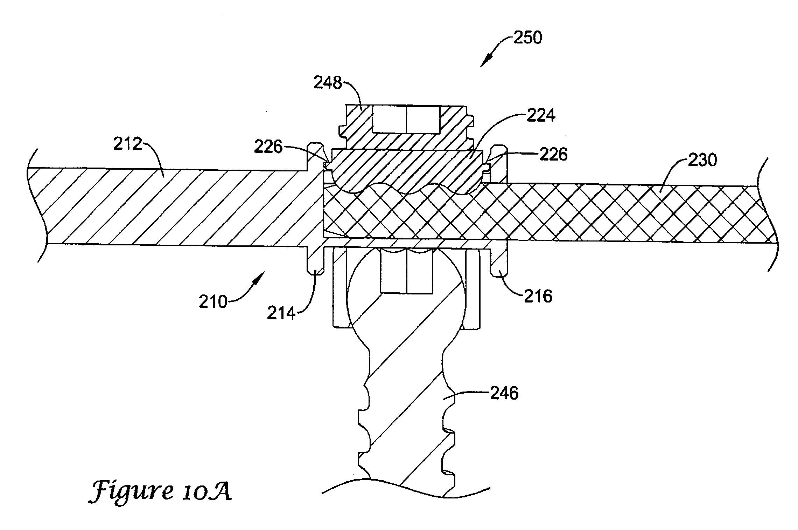

FIGS. 9 and 10 illustrate another vertebral stabilization system 250 including a transition connector 210 for coupling a rigid segment to a flexible segment. The transition connector 210 may include a connection portion 211 and a rigid rod portion 212 extending from the connection portion 211. While the rigid rod portion 212 is shown as having a circular cross-section, the rigid rod portion 212 may have a cross-section of any desired shape. The rigid rod portion 212 may be any desired length, such as a length sufficient to extend between two, three, four, or more vertebrae of the spinal column.

In some examples, the rigid rod portion 212 and the connection portion 211 may be formed as a unitary structure. In other examples, the connection portion 211 may be fixedly attached to the rigid rod portion 212. For example, the connection portion 211 may be welded to the rigid rod portion 212 or may be attached using an adhesive. It is contemplated that if the connection portion 211 is fixedly attached to the rigid rod portion 212, the connection portion 211 and the rigid rod portion 212 may comprise different materials.

The connection portion 211 may include a first flange 24 and a second flange 216 spaced from the first flange 214 by a medial region 218. The medial region 218 may have a cross-sectional dimension less than the cross-sectional dimension of each of the first and second flanges 214, 216. For instance, the medial region 218 may be sized for insertion into the U-shaped channel of the head portion of a vertebral fastener, with the first flange 214 located exterior of the head portion on a first side of the vertebral fastener and the second flange 216 located exterior of the head portion on a second side of the vertebral fastener, as will be further described herein. The first flange 214 may be located proximate the rigid rod portion 212, such that the rigid rod portion 212 extends from the first flange 214.

The connection portion 211 may include a central bore 222 extending from the second flange 216 into the medial region 218. The central bore 222 may be sized to receive a portion of a flexible member, such as an end portion a flexible cord 230. The connection portion 211 may also include a side opening 220 in the medial region 218 opening into the central bore 222 for receiving a cap 224, or other locking member, to secure the flexible cord 230 to the transition connector 210. The cap 224 may include an interlocking structure which mates with complementary interlocking structure of the connection portion 211 to interlock the cap 224 to the medial portion 2 i 8 when the cap 224 is disposed in the side opening 220. For instance, the cap 224 may include one or more tabs and/or grooves which mate and interlock with one or more tabs and/or grooves of the medial portion 218. As shown in the figures, the connection portion 211 may include a tab 226 extending into the side opening 220 from each flange 214, 216 which engage a surface of the cap 224 when the cap 224 is positioned in the side opening 220. It is understood that other interlocking structures may be included instead of or in addition to the interlocking structures shown in the figures.

The vertebral stabilization system 250 may also include a vertebral fastener 240, illustrated as a pedicle screw, having a head portion 242 defining a U-shaped channel 244 for receiving the medial portion 218 of the connection portion 211 therein. Although not shown in FIGS. 9 and 10, it is understood that the vertebral fastener 240 may include a bone engaging portion, such as a threaded shank, extending from the head portion 242 for engagement with a vertebra of a spinal column. A securing member, shown as a threaded fastener 248, may be configured for engagement with the head portion 242 to retain the connection portion 211 of the transition connector 210 in the U-shaped channel 244.

FIG. 10 illustrates the vertebral stabilization system 250 in an assembled configuration, with the medial portion 218 positioned in the U-shaped channel 244 of the head portion 242 of a vertebral fastener 240 and the flanges 216, 218 positioned on either side of the head portion 242. In the assembled configuration, an end portion of the flexible cord 230 is positioned in the central bore 222 of the connection portion 211 and the cap 224 is inserted into the side opening 220 to engage the flexible cord 230.

As can be seen in FIG. 10A, the tabs 226 interlock with the cap 224 to secure the flexible cord 230 to the transition connector 210. The interlocking structures of the cap 224 and the connection portion 211 may be configured such that when the cap 224 is interlocked with the connection portion 211 the cap 224 applies a compressive force against the flexible cord 230 in the bore 222 to restrain the flexible cord 230 from being removed from the transition connector 210. The surface of the cap 224 in contact with the flexible cord 230 may have a contoured surface, one or bumps or protrusions, serrations, grooves, or structural features to increase resistance to removal of the flexible cord 230. Furthermore, when the threaded fastener 248, or other securing member, is engaged with the head portion 242, the threaded fastener 248 may apply a securing force against the cap 224 to further press the cap 224 against the flexible cord 230.

Although not shown in FIG. 10, the rigid rod portion 212 may extend to one or more additional vertebral fasteners which may be secured to vertebrae of the spinal column in a similar fashion as the vertebral fastener 240. Furthermore, the cord 230 may extend in an opposite direction from the transition connector 210 to one or more additional vertebral fasteners which may be secured to vertebrae of the spinal column in a similar fashion as the vertebral fastener 240. Furthermore, a spacer, such as that shown in FIG. 4, may be positioned between the second flange 216 and another vertebral fastener with the flexible cord 230 extending through the spacer, to provide a flexible construct at one or more vertebral levels.

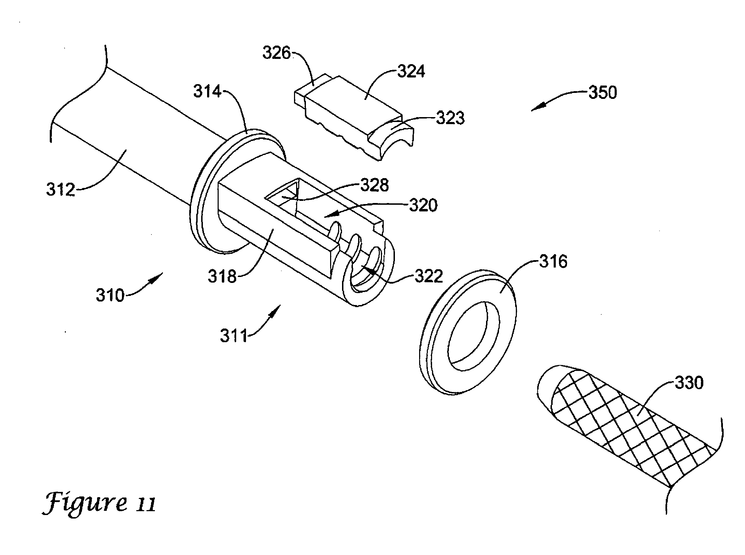

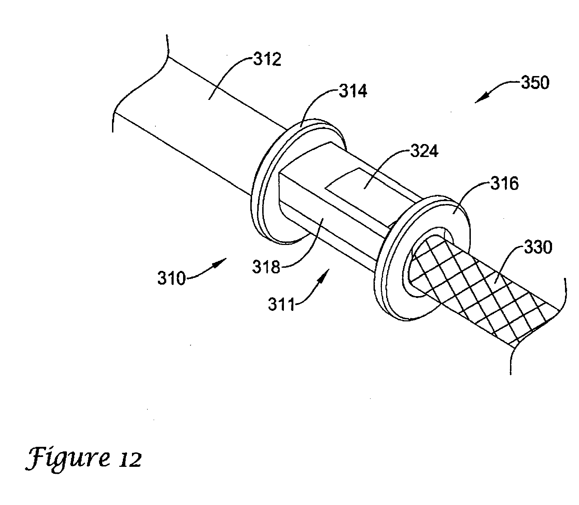

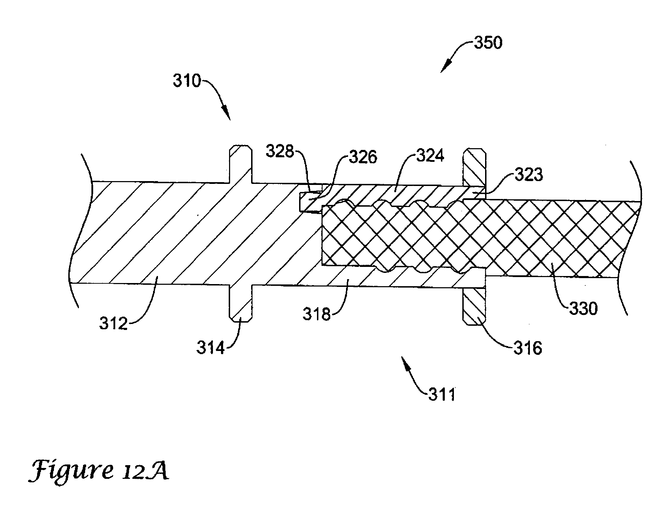

FIGS. 11 and 12 illustrate another vertebral stabilization system 350 including a transition connector 310 for coupling a rigid segment to a flexible segment. The transition connector 310 may include a connection portion 311 and a rigid rod portion 312 extending from the connection portion 311. While the rigid rod portion 312 is shown as having a circular cross-section, the rigid rod portion 312 may have a cross-section of any desired shape. The rigid rod portion 312 may be any desired length, such as a length sufficient to extend between two, three, four, or more vertebrae of the spinal column.

In some examples, the rigid rod portion 312 and the connection portion 311 may be formed as a unitary structure. In other examples, the connection portion 311 may be fixedly attached to the rigid rod portion 312. For example, the connection portion 311 may be welded to the rigid rod portion 312 or may be attached using an adhesive. It is contemplated that if the connection portion 311 is fixedly attached to the rigid rod portion 312, the connection portion 311 and the rigid rod portion 312 may comprise different materials.

The connection portion 311 may include a flange 314 proximate the rigid rod portion 312 and a housing 318 opposite the rigid rod portion 312. The housing 318 may have a cross-sectional dimension less than the cross-sectional dimension of the flange 314. For instance, the housing 318 may be sized for insertion into the U-shaped channel of the head portion of a vertebral fastener, with the flange 314 located exterior of the head portion on a first side of the vertebral fastener, as will be further described herein. The flange 314 may be located proximate the rigid rod portion 312, such that the rigid rod portion 312 extends from the flange 314.

The connection portion 311 may include a central bore 322 extending from an end of the housing 318 into the housing 318. The central bore 322 may be sized to receive a portion of a flexible member, such as an end portion a flexible cord 330. The connection portion 311 may also include a side opening 320 in the housing 318 opening into the central bore 322 for receiving a cap 324 to secure the flexible cord 330 to the transition connector 310. The cap 324 may include an interlocking structure which mates with complementary interlocking structure of the connection portion 311 to interlock the cap 324 to the housing 318 when the cap 324 is disposed in the side opening 320. For instance, the cap 324 may include one or more tabs 326 which fit into one or more recesses 328 of the housing 318. It is understood, however, that other interlocking structures may be included instead of or in addition to the interlocking structures shown in the figures.

The transition connector 310 may also include a retaining ring 316 which may be slidably disposed over the flexible cord 330 up to the housing 318 in order to lock the cap 324 to the housing 318. For example, the retaining ring 316 may circumscribe an end region of the housing 318 and an end region 323 of the cap 324 to restrict decoupling the cap 324 from the housing 318. For example, the end region 323 of the cap 324 and the end region of the housing 318, being collectively cylindrical in shape in the illustrated embodiment, may be disposed in the central opening of the retaining ring 316. The retaining ring 316 may apply a clamping force onto the cap 324 to press the cap 324 into engagement with the flexible cord 330 positioned in the bore 322. In some examples, an inner diameter of the retaining ring 316 may be substantially the same as an outer diameter of the end regions of the housing 318 and cap 324, collectively. In other embodiments, the inner diameter of the retaining ring 316 may be slightly less than the outer diameter of the end regions of the housing 318 and cap 324, collectively, creating a press fit between the components. In some examples, the retaining ring 316 may fit into a groove or channel extending circumferentially end regions of the housing 318 and cap 324.

The retaining ring 316 may be of any shape desired such that an inner surface of the ring 316 is configured to engage the end regions of the housing 318 and cap 324 and maintain the cap 324 secured against the flexible cord 330. In some embodiments, the retaining ring 316 may be a discontinuous ring, such as a C-shaped ring, which can be expanded and/or contracted around the end regions of the housing 318 and cap 324. For instance, the retaining ring 316 may be crimped around the end regions to secure the cap 324. In some embodiments, once the retaining ring 316 is disposed around the end regions, the retaining ring 316 may be fixedly secured to the housing 318 and/or cap 324. The retaining ring 316 may, for example, be adhesively bonded or welded to the housing 318 and/or cap 324.

Although not shown in FIGS. 11 and 12, it is understood that the vertebral stabilization system 350 may also include one or more vertebral fasteners, such as pedicle screws, as disclosed herein. For instance, the vertebral fasteners may include a bone engaging portion, such as a threaded shank, extending from a head portion for engagement with a vertebra of a spinal column. A securing member, such as a threaded fastener, may be configured for engagement with the head portion to retain the connection portion 311 of the transition connector 310 in the U-shaped channel of the head portion, with the flange 314 positioned on one side of the head portion and the retaining ring 316 positioned on the opposite side of the head portion of the vertebral fastener.

FIG. 12 illustrates the vertebral stabilization system 350 in an assembled configuration. In the assembled configuration, an end portion of the flexible cord 330 is positioned in the central bore 322 of the connection portion 311, the cap is inserted into the side opening 320 to engage the flexible cord 330, and the retaining ring 316 is positioned around and end portion of the housing 318 and the cap 324 to hold the cap 324 into engagement with the flexible cord 330.

As can be seen in FIG. 12A, the tab 326 of the cap 324 is positioned in the recess 328 of the housing 318 and the retaining ring 316 is positioned over the end regions of the housing 318 and cap 324 to secure the flexible cord 330 to the transition connector 310. The interaction of the cap 324 with the housing 318 and the retaining ring 316 may be configured such that when the cap 324 is secured to the housing 318, the cap 324 applies a compressive force against the flexible cord 330 in the bore 322 to restrain the flexible cord 330 from being removed from the transition connector 310. The surface of the cap 324 in contact with the flexible cord 330 may have a contoured surface, one or bumps or protrusions, serrations, grooves, or structural features to increase resistant to removal of the flexible cord 330. Furthermore, when a threaded fastener, or other securing member, is engaged with the head portion of the vertebral fastener into which the connection portion 311 is positioned, the threaded fastener may apply a securing force against the cap 324 to further press the cap 324 against the flexible cord 330.

Although not shown in FIG. 12, the rigid rod portion 312 may extend to one or more additional vertebral fasteners which may be secured to vertebrae of the spinal column. Furthermore, the cord 330 may extend in an opposite direction from the rigid rod portion 312 from the transition connector 310 to one or more additional vertebral fasteners which may be secured to vertebrae of the spinal column. Furthermore, a spacer, such as that shown in FIG. 4, may be positioned between the retaining ring 316 and another vertebral fastener with the flexible cord 330 extending through the spacer, to provide a flexible construct at one or more vertebral levels.

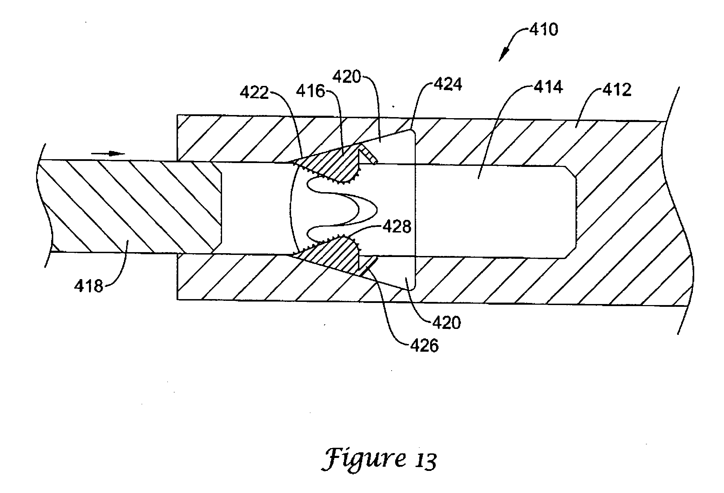

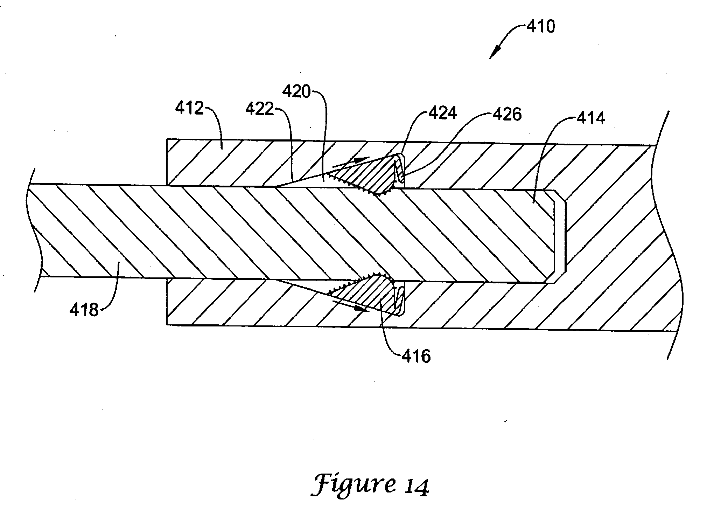

FIG. 13 and FIG. 14 illustrate an alternative example of an illustrative transition connector 410. The connector 410 may comprise a rigid rod portion 412. The rigid rod portion 412 may have a cross section of any desired shape, including, but not limited to: circular, square, rectangular, polygonal, or elliptical. The rigid rod portion 412 may be of any length necessary to extend between two, three, four, or more vertebrae of the spinal column. The rigid rod portion 412 may further comprise a bore 414 for receiving a flexible cord 418, or other flexible member. The bore 414 may comprise an area 420 of increased diameter relative to the diameter of other portions of the bore 414. The area 420 of increased diameter may have a first end 422 having a first diameter and a second end 424 having a larger diameter than the first end 422. The first end 422 may be located closer to the open end of the bore 414 than the second end 424. A compressible and/or expandable retainer ring 416 may be disposed within the area 420 near the first end 422. The retaining ring 416 may include biasing tabs 426. The biasing tabs 426 may be configured to bias the retaining ring 416 towards the first end 422 of the area 420 of increased diameter. The retaining ring 416 may further comprise surface roughenings 428 which may help maintain the cord 418 within the bore 414. The surface roughenings 428 may include any mechanical gripping means such as, but not limited to, one or more threads, ribs, projecting grooves, teeth, and/or serrations or combination thereof.

The cord 418 may be longitudinally moved into the bore 414 along a central longitudinal axis in the direction indicated by the arrow in FIG. 13. As the end of the cord 418 engages the retainer ring 416, the retainer ring 416 may move towards the second end 424 of the enlarged area 420 as indicated by the arrows in FIG. 14, allowing the inner periphery of the retainer ring 416 to be enlarged to allow the cord 418 to pass through the retainer ring 416. In some examples, the retainer ring 416 may be sized such that even when it is disposed at the second end 424 of the enlarged area 420 it extends into the bore 414. The retainer ring 416 may have a constant cross-sectional area or may have a generally increasing or generally decreasing cross sectional area. The retainer ring 416 may extend into the bore 414 such that it may apply a radially compressive force on the cord 418 which may prevent the cord 418 from being retracted from the bore 414.

As can be seen in FIG. 14, the biasing tabs 426 may contact the end surface of the second end 424 of the enlarged portion 420 of the bore 414. When pressure is being applied to the retainer ring 416 as the cord 418 is being advanced into the bore 414, the biasing tabs 426 may compress between the retainer ring 416 and the end surface of the second end 424 of the enlarged portion 420. Once the cord 418 has been advanced to its desired location, the pressure on the biasing tabs 426 may be relieved. This may allow the biasing tabs 426 to push the retainer ring 416 towards the first end 422. As the retainer ring 416 is biased towards the first end 422, the configuration of the enlarged portion 420 and/or the retainer ring 416 may bias the retainer ring 416 to move radially inward further into the bore 414 causing the retainer ring 416 to apply more force to the cord 418. Similarly, if an attempt is made to retract the cord 418 from the bore 414 once it has passed the retainer ring 416, the configuration of the enlarged portion 420 and/or the retainer ring 416 may cause the retainer ring 416 to place an even larger compressive force on the cord 418 than at the second end 424 of the area 420 as the retainer ring 416 is urged further radially inward into the bore 414, locking the cord 418 in the bore 414 of the rod 412.

A method, not claimed, of securing a flexible member of a vertebral stabilization system to a rigid member of the flexible stabilization system may comprise
providing a rigid rod having an end portion including a clam shell connector, the clam shell connector including a first segment expending from an enlarged diameter portion of the rigid rod to a free end of the first segment and a second segment extending from the enlarged diameter portion of the rigid rod to a free end of the second segment, the free end of the first segment being discontinuous with the free end of the second segment;
wherein the rigid rod is provided with the clam shell connector in an open position in which the free end of the first segment is spread apart and spaced from the free end of the second segment;
positioning an end portion of a flexible member in the clam shell connector between the first segment and the second segment;
exerting a clamping force on the first segment and/or the second segment to urge the clam shell connector to a closed position in which the end portion of the flexible member is clamped between the first segment and the second segment.

Said method may further comprise
placing a retaining ring around the clam shell connector proximate the free end of the first segment and the free end of the second segment to for prevent the first segment of the clam shell connector from spreading apart from the second segment of the clam shell connector.

Said method may further comprise:
positioning the clam shell connector in a channel of a head portion of a vertebral anchor; and
threadably engaging a threaded fastener with a threaded opening of the head portion of the vertebral anchor to secure the clam shell connector in the channel.

It may be envisaged that the threaded fastener applies a further clamping force to the clam shell connector to prevent the first segment and the second segment from spreading apart.

A vertebral stabilization system, not claimed, may comprise:
a flexible member;
a rigid rod having an end portion including a bore configured to receive an end portion of the flexible member;
wherein the bore further comprises an area of increased diameter having a first end having a first diameter and a second end having a second diameter greater than the first diameter; and
a retainer ring slidably disposed within the area of increased diameter;
wherein the retainer ring is configured to slide from the first end toward the second end as the portion of the flexible member is advanced into the bore; and
wherein the retainer ring is biased toward the first end to maintain a radially compressive force on the flexible member.

Said vertebral stabilization system may be designed such that the retainer ring includes one or more biasing members engaged against an end wall of the area of increased diameter to bias the retainer ring toward the first end.

Said vertebral stabilization system may further be designed such that the first end of the area of increased diameter is located closer to an open end of the bore than the second end of the area of increased diameter.

## Claims

1. A vertebral stabilization system comprising:
a flexible member (30, 134); and
a rigid rod (12, 112) having an end portion including a clam shell connector (14, 114) configured to receive an end portion of the flexible member (30, 134);
the clam shell connector (14, 114) formed as a unitary portion of the rigid rod (12, 112) extending from an enlarged diameter portion of the rigid rod (12, 112), the clam shell connector (14, 114) including a first segment (16, 116) and a second segment (18, 118);
wherein the first segment (16, 116) extends from the enlarged diameter portion of the rigid rod (12, 112) to a free end (24, 124) of the first segment (16, 116), and the second segment (18, 118) extends from the enlarged diameter portion of the rigid rod (12, 112) to a free end (26, 126) of the second segment (18, 118);
wherein the free end (24, 124) of the first segment (16, 116) is discontinuous with the free end (26, 126) of the second segment (18, 118);
further **characterised in that**:
the flexible member (30, 134) is a card, and
**in that** the free end (24, 124) of the first segment (16, 116) contacts the free end (26, 126) of the second segment (18, 118) when an end portion of the card (30, 134) is clamped between the first segment (16, 116) and the second segment (18, 118) of the clam shell connector (14, 114).

2. The vertebral stabilization system of claim 1, further comprising:
a retaining ring (32) positioned around the clam shell connector (14) for preventing the first segment (16) of the clam shell connector (14) from spreading apart from the second segment (18) of the clam shell connector (14).

3. The vertebral stabilization system of claim 2, further comprising:
a vertebral fastener (34, 136) including a head portion (42, 140) and a shank (40, 138) extending from the head portion (42, 140), the head portion (42, 140) of the vertebral fastener (34, 136) including a channel (142) extending from a first side surface of the head portion (42, 140) to a second side surface of the head portion (42, 140);
wherein the clam shell connector (14, 114) of the rigid rod (12, 112) is positioned in the channel (142) of the head portion (42, 140) of the vertebral fastener (34, 136).

4. The vertebral stabilization system of claim 3, wherein the enlarged diameter portion of the rigid rod (12, 112) is positioned adjacent the first side surface of the head portion (42, 140) of the vertebral fastener (34, 136) and the retaining ring (32) is positioned adjacent the second side surface of the head portion (42, 140) of the vertebral fastener (34, 136).

5. The vertebral stabilization system of claim 4, further comprising a spacer (46) having a lumen extending from a first end surface of the spacer (46) to a second end surface of the spacer (46), wherein the flexible member (30, 134) extends through the lumen of the spacer (46) such that the first end surface of the spacer (46) faces the retaining ring (32).

6. The vertebral stabilization system of any preceding claim, wherein a surface of the first segment (16, 116) in contact with the flexible member (30, 134) and/or a surface of the second segment (18, 118) in contact with the flexible member (30, 134) includes surface roughenings.

7. The vertebral stabilization system of claim 6, wherein the surface roughenings include one or more threads or teeth.

8. The vertebral stabilization system of claim 6, wherein the surface roughenings include one or more ribs or grooves.

9. The vertebral stabilization system of claim 6, wherein the surface roughenings include one or more serrations.

10. The vertebral stabilization system of any preceding claim, wherein the first segment (16, 116) is an upper half of the clam shell connector (14, 114) and the second segment (18, 118) is a lower half of the clam shell connector (14, 114).

11. The vertebral stabilization system of claim 2, wherein the retaining ring (32) is adhesively bonded to the clam shell connector (14).

12. The vertebral stabilization system of claim 2, wherein the retaining ring (32) is welded to the clam shell connector (14).

13. The vertebral stabilization system of claim 2, wherein the retaining ring (32) is disposed in a groove extending around the clam shell connector (14).

14. The vertebral stabilization system of claim 2, wherein the retaining ring (32) includes a tapered surface which mates with a tapered surface of the clam shell connector (14).

15. The vertebral stabilization system of any preceding claim, wherein the flexible member (30, 134) is a cord.

## Patentansprüche

1. Wirbelstabilisierungssystem, umfassend:
ein flexibles Element (30, 134); und
eine starre Stange (12, 112) mit einem Endabschnitt, der einen Greiferverbinder (14, 114) aufweist, der so konfiguriert ist, einen Endabschnitt des flexiblen Elements (30, 134) aufzunehmen;
wobei der Greiferverbinder (14, 114) als ein einheitlicher Abschnitt der starren Stange (12, 112) geformt ist und sich von einem Abschnitt mit vergrößertem Durchmesser der starren Stange (12, 112) erstreckt, wobei der Greiferverbinder (14, 114) ein erstes Segment (16, 116) und ein zweites Segment (18, 118) aufweist;
wobei sich das erste Segment (16, 116) von dem Abschnitt mit vergrößertem Durchmesser der starren Stange (12, 112) zu einem freien Ende (24, 124) des ersten Segments (16, 116) erstreckt und sich das zweite Segment (18, 118) von dem Abschnitt mit vergrößertem Durchmesser der starren Stange (12, 112) zu einem freien Ende (26, 126) des zweiten Segments (18, 118) erstreckt;
wobei das freie Ende (24, 124) des ersten Segments (16, 116) von dem freien Ende (26, 126) des zweiten Segments (18, 118) unterbrochen ist;
ferner **dadurch gekennzeichnet, dass** das flexible Element (30, 134) ein Kord ist, und dass das freie Ende (24, 124) des ersten Segments (16, 116) mit dem freien Ende (26, 126) des zweiten Segments (18, 118) in Kontakt steht, wenn ein Endabschnitt des Kords (30, 134) zwischen das erste Segment (16, 116) und das zweite Segment (18, 118) des Greiferverbinders (14, 114) geklemmt ist.

2. Wirbelstabilisierungssystem nach Anspruch 1, ferner umfassend:
einen Haltering (32), der um den Greiferverbinder (14) positioniert ist, um zu verhindern, dass sich das erste Segment (16) des Greiferverbinders (14) von dem zweiten Segment (18) des Greiferverbinders (14) abspreizt.

3. Wirbelstabilisierungssystem nach Anspruch 2, ferner umfassend:
eine Wirbelbefestigungseinrichtung (34, 136), die einen Kopfabschnitt (42, 140) und einen Schaft (40, 138) aufweist, der sich von dem Kopfabschnitt (42, 140) erstreckt, wobei der Kopfabschnitt (42, 140) der Wirbelbefestigungseinrichtung (34, 136) einen Kanal (142) aufweist, der sich von einer ersten Seitenfläche des Kopfabschnitts (42, 140) zu einer zweiten Seitenfläche des Kopfabschnitts (42, 140) erstreckt;
wobei der Greiferverbinder (14, 114) der starren Stange (12, 112) in dem Kanal (142) des Kopfabschnitts (42, 140) der Wirbelbefestigungseinrichtung (34, 136) positioniert ist.

4. Wirbelstabilisierungssystem nach Anspruch 3, wobei der Abschnitt mit vergrößertem Durchmesser der starren Stange (12, 112) benachbart der ersten Seitenfläche des Kopfabschnitts (42, 140) der Wirbelbefestigungseinrichtung (34, 136) positioniert ist und der Haltering (32) benachbart der zweiten Seitenfläche des Kopfabschnitts (42, 140) der Wirbelbefestigungseinrichtung (34, 136) positioniert ist.

5. Wirbelstabilisierungssystem nach Anspruch 4, ferner mit einem Abstandhalter (46), der ein Lumen aufweist, das sich von einer ersten Endfläche des Abstandhalters (46) zu einer zweiten Endfläche des Abstandhalters (46) erstreckt, wobei sich das flexible Element (30, 134) durch das Lumen des Abstandhalters (46) erstreckt, so dass die erste Endfläche des Abstandhalters (46) zu dem Haltering (32) weist.

6. Wirbelstabilisierungssystem nach einem der vorhergehenden Ansprüche, wobei eine Fläche des ersten Segments (16, 116) in Kontakt mit dem flexiblen Element (30, 134) und/oder eine Fläche des zweiten Segments (18, 118) in Kontakt mit dem flexiblen Element (30, 134) Oberflächenaufrauungen umfasst.

7. Wirbelstabilisierungssystem nach Anspruch 6, wobei die Oberflächenaufrauungen ein oder mehrere Gewinde oder Zähne umfassen.

8. Wirbelstabilisierungssystem nach Anspruch 6, wobei die Oberflächenaufrauungen eine oder mehrere Rippen oder Nuten umfassen.

9. Wirbelstabilisierungssystem nach Anspruch 6, wobei die Oberflächenaufrauungen eine oder mehrere Kerbverzahnungen umfassen.

10. Wirbelstabilisierungssystem nach einem der vorhergehenden Ansprüche, wobei das erste Segment (16, 116) eine obere Hälfte des Greiferverbinders (14, 114) ist und das zweite Segment (18, 118) eine untere Hälfte des Greiferverbinders (14, 114) ist.

11. Wirbelstabilisierungssystem nach Anspruch 2, wobei der Haltering (32) an den Greiferverbinder (14) geklebt ist.

12. Wirbelstabilisierungssystem nach Anspruch 2, wobei der Haltering (32) an den Greiferverbinder (14) geschweißt ist.

13. Wirbelstabilisierungssystem nach Anspruch 2, wobei der Haltering (32) in einer Nut angeordnet ist, die sich um den Greiferverbinder (14) erstreckt.

14. Wirbelstabilisierungssystem nach Anspruch 2, wobei der Haltering (32) eine konische Fläche aufweist, die mit einer konischen Fläche des Greiferverbinders (14) zusammenpasst.

15. Wirbelstabilisierungssystem nach einem der vorhergehenden Ansprüche, wobei das flexible Element (30, 134) ein Kord ist.

## Revendications

1. Système de stabilisation vertébrale comprenant :
un élément flexible (30, 134) ; et
une barre rigide (12, 112) ayant une portion terminale incluant un connecteur à coque pincée (14, 114) configuré pour recevoir une portion terminale de l'élément flexible (30, 134) ;
le connecteur à coque pincée (14, 114), formé comme une portion unitaire de la barre rigide (12, 112) et s'étendant depuis une portion à diamètre élargi de la barre rigide (12, 112), le connecteur à coque pincée (14, 114) incluant un premier segment (16, 116) et un second segment (18, 118) ;
dans lequel le premier segment (16, 116) s'étend depuis la portion à diamètre élargi de la barre rigide (12, 112) vers une extrémité libre (24, 124) du premier segment (16, 116), et le second segment (18, 118) s'étend depuis la portion à diamètre élargi de la barre rigide (12, 112), une extrémité libre (26, 126) du second segment (18, 118) ;
dans lequel l'extrémité libre (24, 124) du premier segment (16, 116) est discontinue par rapport à l'extrémité libre (26, 126) du second segment (18, 118) ;
**caractérisé en outre en ce que** :
l'élément flexible (30, 134) est un cordon, et
**en ce que** l'extrémité libre (24, 124) du premier segment (16, 116) vient en contact avec l'extrémité libre (26, 126) du second segment (18, 118) quand une portion terminale du cordon (30, 134) est pincée entre le premier segment (16, 116) et le second segment (18, 118) du connecteur à coque pincée (14, 114).

2. Système de stabilisation vertébrale selon la revendication 1, comprenant en outre :
une bague de retenue (32) positionnée autour du connecteur à coque pincée (14) pour empêcher au premier segment (16) du connecteur à coque pincée (14) de s'écarter du second segment (18) du connecteur à coque pincée (14).

3. Système de stabilisation vertébrale selon la revendication 2, comprenant en outre :
un fixateur vertébral (34, 135) incluant une portion de tête (42, 140) et une tige (40, 138) s'étendant depuis la portion de tête (42, 140), la portion de tête (42, 140) du fixateur vertébral (34, 136) incluant un canal (142) s'étendant depuis une première surface latérale de la portion de tête (42, 140) vers une second surface latérale de la portion de tête (42, 140) ;
dans lequel le connecteur à coque pincée (14, 114) de la barre rigide (12, 112) est positionné dans le canal (142) de la portion de tête (42, 140) du fixateur vertébral (34, 136).

4. Système de stabilisation vertébrale selon la revendication 3, dans lequel la portion à diamètre élargi de la barre rigide (12, 112) est positionnée adjacente à la première surface latérale de la portion de tête (42, 140) du fixateur vertébral (34, 136) et la bague de retenue (32) est positionnée adjacente à la seconde surface latérale de la portion de tête (42, 140) du fixateur vertébral (34, 136).

5. Système de stabilisation vertébrale selon la revendication 4, comprenant en outre un élément d'espacement (46) ayant un lumen s'étendant depuis une première surface terminale de l'élément d'espacement (46) jusqu'à une seconde surface terminale de l'élément d'espacement (46), dans lequel l'élément flexible (30, 134) s'étend à travers le lumen de l'élément d'espacement (46) de telle façon que la première surface terminale de l'élément d'espacement (46) fait face vers la bague de retenue (32).

6. Système de stabilisation vertébrale selon l'une quelconque des revendications précédentes, dans lequel une surface du premier segment (16, 116) en contact avec l'élément flexible (30, 134) et/ou une surface du second segment (18, 118) en contact avec l'élément flexible (30, 134) inclut des rugosités de surface.

7. Système de stabilisation vertébrale selon la revendication 6, dans lequel les rugosités de surface incluent un ou plusieurs filets ou une ou plusieurs dents.

8. Système de stabilisation vertébrale selon la revendication 6, dans lequel les rugosités de surface incluent une ou plusieurs nervures ou gorges.

9. Système de stabilisation verticale selon la revendication 6, dans lequel les rugosités de surfaces incluent une ou plusieurs cannelures.

10. Système de stabilisation verticale selon l'une quelconque des revendications précédentes, dans lequel le premier segment (16, 116) est une moitié supérieure du connecteur à coque pincée (14, 114) et le second segment (18, 118) est une moitié inférieure du connecteur à coque pincée (14, 114).

11. Système de stabilisation verticale selon la revendication 2, dans lequel la bague de retenue (32) est jointe de manière adhésive au connecteur accord passé (14).

12. Système de stabilisation vertébrale selon la revendication 2, dans lequel la bague de retenue (32) est soudée au connecteur à coque pincée (14).

13. Système de stabilisation vertébrale selon la revendication 2, dans lequel la bague de retenue (32) est disposée dans une gorge s'étendant autour du connecteur à coque pincée (14).

14. Système de stabilisation vertébrale selon la revendication 2, dans lequel la bague de retenue (32) inclut une surface effilée qui est appariée avec une surface effilée du connecteur à coque pincée (14).

15. Système de stabilisation vertébrale selon l'une quelconque des revendications précédentes, dans lequel l'élément flexible (30, 134) est un cordon.
